# EUROPÄISCHE PATENTANMELDUNG

(11) **EP 1 939 177 A2**
(43) Veröffentlichungstag der Anmeldung: **02.07.2008**
(21) Anmeldenummer: 07123005.6
(22) Anmeldetag: 12.12.2007
(51) Int. Cl.: C07D 211/02, C07D 211/26

(54) **Verfahren zur Herstellung von 2-Aminomethylpiperidin**

(30) Priorität: 27.12.2006 DE 102006061533
(71) Anmelder: Saltigo GmbH, 40764 Langenfeld (DE)
(72) Erfinder: Antons, Stefan, 51373, Leverkusen (DE); Dreisbach, Claus, 42799, Leichlingen (DE)
(74) Vertreter: Pettrich, Klaus-Günter

(57) **Zusammenfassung**

Die Erfindung betrifft ein Verfahren zur Herstellung von Aminomethylpyridinen der allgemeinen Formel (I) 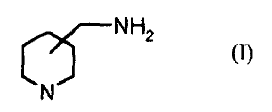 durch Hydrierung von Cyanopyridinen der allgemeinen Formel (II) 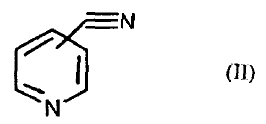 mit Wasserstoff unter erhöhtem Druck gegebenenfalls in Gegenwart eines Ni-, Fe- oder Co-haltigen Katalysators und gegebenenfalls in Gegenwart von Ammoniak.

## Beschreibung

Die Erfindung betrifft die Herstellung von 2-Aminomethylpiperidin.

2-Aminomethylpiperidin stellt einen wesentlichen Baustein zur Herstellung von pharmazeutischen Wirkstoffen dar. Einer dieser pharmazeutischen Wirkstoffe ist Flecainide

Die Herstellung von Flecainide ist z.B. in Chem. Ber.; GE; 118; 11; 1985; 4616-4619 und in J. Pharm. Sci.; EN; 80; 9; 1991; 887-890 beschrieben.

Es hat nicht an Versuchen gefehlt den Baustein 2-Aminomethylpiperidin auf möglichst einfache und wirtschaftliche Weise herzustellen. So ist in JACS (1941), S 490 und JACS (1946) S. 1330 die Herstellung ausgehend von 2-Cyanopyridin beschrieben. Jedoch ist das beschrieben Verfahren zweistufig, die gesamte Ausbeute beträgt nur etwas über 20 % d. Th. Weitere schwerwiegende Nachteile der beschrieben Vorgehensweise bestehen in der Verwendung sehr teurer Edelmetallkatalysatoren wie z. B .P. Auch wird der zweite Reduktionsschritt in Essigsäure oder anderen Mineralsäuren durchgeführt. Dies hat neben der möglichen Gefahr von Materialkorrosion den großen Nachteil, das sehr viel zusätzlicher Aufwand erforderlich ist, das gut wasserlösliche 2-Aminomethylpiperidin aus seinen Salzen zu isolieren. Die Verwendung von 2-Aminomethylpyridin zur Herstellung des 2-Aminomethylpiperidins ist in Tetrahedron Asymetry (1998, S. 1597); und in J.Pharm. Sci. (1990, S 750-53) beschrieben. Jedoch sind auch hier die oben beschrieben Probleme und Verfahrensnachteile immer noch vorhanden.

Es bestand daher die Aufgabe, ein technisch durchführbares und wirtschaftliches Verfahren zu finden, das es auf einfache Weise ermöglicht, 2-Aminomethylpiperidin im industriellen Maßstab herzustellen.

Überraschenderweise wurde nun gefunden, dass es möglich ist, ausgehend von 2-Cyanopyridin in einem Schritt zum 2-Aminomethylpiperidin zu gelangen. Auch wurde gefunden, dass die Umsetzung auf einfache Weise ohne die Verwendung teurer Edelmetall-Katalysatoren und zusätzlicher Säuren sowie ohne den Einsatz eines zusätzlichen Lösungsmittels möglich ist.

Gegenstand der Erfindung ist daher ein Verfahren zur Herstellung von Aminomethylpyridinen der allgemeinen Formel (I) 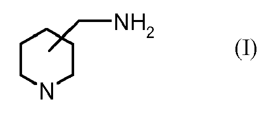 durch Hydrierung von Cyanopyridinen der allgemeinen Formel (II) 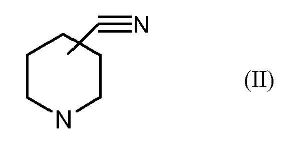 mit Wasserstoff unter erhöhtem Druck, gegebenenfalls in Gegenwart eines Ni-, Fe- oder Co-haltigen Katalysators und gegebenenfalls in Gegenwart von Ammoniak.

Hierdurch wird eine sehr effiziente und wirtschaftliche Herstellung von 2-Aminomethylpiperidin möglich, indem 2-Cyanopyridin in Substanz gegebenenfalls in Gegenwart von Nickel und/oder Kobalt-haltigen Katalysatoren direkt zum 2-Aminomethylpiperidin hydriert wird. Nach Abfiltrieren des Katalysators wird das Rohprodukt destilliert, so dass reines 2-Aminomethylpiperidin erhalten wird.

Bevorzugt wird bei 100 bis 200°C und einem Druck von 50 bis 300 bar gearbeitet.

### Beispiele:

### Beispiel 1 ausgehend von 2-Aminomethylpyridin

Eine Mischung aus 5 g Ra-Ni 5584 und 100 g 2-Aminomethylpyridin wurden mit 20 bar Wasserstoff versetzt und anschließend auf 160 °C erwärmt der Wasserstoffdruck wird auf 180 bar erhöht und anschließend bis zum Ende der Wasserstoffaufnahme hydriert. Nach Abkühlen und Entspannen wurde mit Stickstoff belüftet und der Katalysator abfiltriert. Das erhaltene Rohprodukt zeigte einen Gehalt von 97 % 2-Aminomethylpiperidin.

### Beispiel 2 ausgehend von 2-Aminomethylpyridin

Eine Mischung aus 10 g Ra-Ni 5584 und 100 g 2-Aminomethylpyridin wurde mit 20 bar Wasserstoff versetzt und anschließend auf 200 °C erwärmt der Wasserstoffdruck wurde auf 160 bar erhöht und anschließend bis zum Ende der Wasserstoffaufnahme hydriert. Nach Abkühlen und Entspannen wurde mit Stickstoff belüftet und der Katalysator abfiltriert. Das erhaltene Rohprodukt zeigte einen Gehalt von 94,5 % 2-Aminomethylpiperidin

### Beispiel 3 ausgehend von 2-Aminomethylpyridin

Eine Mischung aus 5 g Ni-Fe-6606 und 100 g 2-Aminomethylpyridin wurde mit 20 bar Wasserstoff versetzt und anschließend auf 160 °C erwärmt der Wasserstoffdruck wurde auf 200 bar erhöht und anschließend bis zum Ende der Wasserstoffaufnahme hydriert. Nach Abkühlen und Entspannen wurde mit Stickstoff belüftet und der Katalysator abfiltriert. Das erhaltene Rohprodukt zeigte einen Gehalt von 67,5 2-Aminomethylpiperidin.

### Beispiel 4 ausgehend von 2-Cyanopyridin

Eine Mischung aus 25 g Ni-5584 und 127,5 g flüssigen Ammoniak wurden mit 100 bar Wasserstoff versetzt und auf 180 °C erhitzt, nach Erhöhung des Wasserstoffdruckes auf 180 bar wurden innerhalb von 6 Stunden 250 g 2-Cyanopyridin in 125 g Ethanol zugegeben. Nach beendeter Wasserstoffaufnahme wurde abgekühlt, entspannt, mit Stickstoff belüftet und der Katalysator abfiltriert. Das erhaltene Rohprodukt zeigte einen Gehalt von 52 % an 2-Aminomethylpiperidin.

## Patentansprüche

1. Verfahren zur Herstellung von Aminomethylpyridinen der allgemeinen Formel (I) 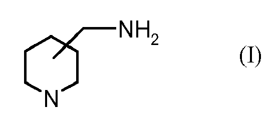 durch Hydrierung von Cyanopyridinen der allgemeinen Formel (II) 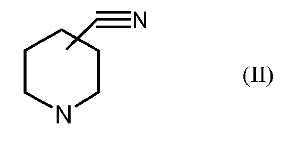 mit Wasserstoff unter erhöhtem Druck, gegebenenfalls in Gegenwart eines Ni-, Fe- oder Co-haltigen Katalysators und gegebenenfalls in Gegenwart von Ammoniak.

2. Verfahren zur Herstellung von Aminomethylpyridinen nach Anspruch 1, **dadurch gekennzeichnet, dass** die Temperatur im Bereich von 10 bis 200°C und der Druck im Bereich von 50 bis 300 bar liegt.

3. Verfahren zur Herstellung von Aminomethylpyridinen nach Anspruch 1, **dadurch gekennzeichnet, dass** Ni, Fe- oder Co-haltigen Katalysatoren eingesetzt werden.
